# EUROPEAN PATENT APPLICATION

(11) **EP 4 818 306 A1**
(43) Date of publication of application: **07.10.2026**
(21) Application number: 24895583.3
(22) Date of filing: 21.06.2024
(51) Int. Cl.: C12N 1/20, A61K 35/745, A61P 3/04, A61P 3/10, A23L 33/135, C12R 1/01

(54) **BIFIDOBACTERIUM ANIMALIS SUBSP. LACTIS STRAIN, PROBIOTIC PREPARATION, AND PREPARATION METHOD THEREFOR AND USE THEREOF**

(30) Priority: 30.11.2023 CN 202311630313; 30.11.2023 CN 202311633450
(71) Applicant: COREE Company Limited, Hong Kong 999077 (HK)
(72) Inventor: WANG, Tingting, Beijing 101300 (CN); ZHANG, Shiqi, Beijing 101300 (CN); ZHANG, Di, Beijing 101300 (CN); ZHANG, Xiao, Beijing 101300 (CN); FAN, Linlin, Beijing 101300 (CN); ZHANG, Zhengwen, Beijing 101300 (CN); XU, Ying, Beijing 101300 (CN); LEE, Soowon, Beijing 101300 (CN); LIM, Chongyoon, Beijing 101300 (CN)
(74) Representative: Studio Torta S.p.A.
(86) International application number: PCT/CN2024/100601
(87) International publication number: WO 2025/112480

(57) **Abstract**

The present invention relates to the technical field of microorganisms, in particular to a *Bifidobacterium animalis* subsp. *lacti* strain, a probiotic preparation and a method for preparing the same and use thereof. The deposit number of *B. animalis* subsp. *lacti* strain is CGMCC No. 25680. The strain and its viable bacteria preparation or dead bacteria preparation can inhibit the differentiation of 3T3-L1 preadipocytes, thereby achieving the effect of effectively inhibiting fat deposition, having the function of weight loss; having the activity of inhibiting DPP-4 and α-glucosidase, and can stimulate NCI-H716 cells to secrete a high level of GLP-1, thereby having the assisting effect of lowering blood glucose.

## Description

### CROSS-REFERENCE TO RELATED APPLICATIONS

This application claims priority to Chinese Patent Application No. 202311630313.0 filed in China on November 30, 2023, and Chinese Patent Application No. 202311633450.X filed in China on November 30, 2023, the entire contents of which are incorporated herein by reference.

### TECHNICAL FIELD

The present invention relates to the technical field of microorganisms, in particular to a *Bifidobacterium animalis* subsp*. lacti* strain, a probiotic preparation and a preparation method therefore and use thereof.

### BACKGROUND

In China, with the rapid development of economy, changes in people's dietary habits and reduced physical activity have led to the earlier onset of obesity-related diseases such as diabetes. Diabetes is a metabolic disease characterized by hyperglycemia. Hyperglycemia is caused by insulin secretion deficiency or impaired biological effects of the insulin, or both. Diabetes can cause vascular and neuropathic lesions, leading to various complications that seriously threatening the health and life of patients.

At present, commercially available hypoglycemic drugs have several common side effects. First, it is hypoglycemia. If the hypoglycemic drug is applied at an excessive dose, or the diet is irregular, it is easy to cause hypoglycemia, especially with insulin secretagogues, including sulfonylureas and glinides, which exert their hypoglycemic effect by promoting insulin, hypoglycemia may occur when used alone or in combination, and in severe cases, hypoglycemic coma may occur. Second, it is gastrointestinal reactions. Oral hypoglycemic drugs may cause gastrointestinal reactions (such as nausea, vomiting, diarrhea, and abdominal distension), like biguanide preparations and glycosidase inhibitors have gastrointestinal reactions. Third, it can damage the liver and kidney. Oral hypoglycemic drugs may aggravate liver and kidney damage, especially in patients with liver and kidney insufficiency. Insulin has no affect liver and kidney function. Fourth, it is drug allergies. Commonly used hypoglycemic drugs may cause allergies, such as skin rash and skin itching. Fifth, other side effects. Such as thiazolidinediones can cause water-sodium retention resulting in edema, while sodium-glucose cotransporter 2 inhibitors may cause genitourinary system infection, ketoacidosis, etc.

*Bifidobacterium animalis* (*B. animalis*) is divided into two subspecies, which are *Bifidobacterium animalis* subsp*. animalis* and *Bifidobacterium animalis* subsp*. lactis*. It controls blood sugar by maintaining intestinal microecological balance, regulating immune responses, reducing oxidative stress, and increasing the secretion of short-chain fatty acids. It possesses good safety and a long history of safe use, and has been listed in the "List of Microbial Strains that Can Be Used in Food" in China and "qualified presumption of safety" (QPS). The 25th document of the Ministry of Health specifies 9 strains of bacteria permitted for use in infants and young children, wherein *Bifidobacterium* species are *B. animalis* subsp*. lactis* Bb-12, *B. animalis* subsp*. lactis* Bi07 and HN019. At present, no excellent *B. animalis* subsp*. lactis* has been screened in China.

Therefore, it is very important to screen an excellent *B. animalis* subsp*. lactis* native to China that is conducive to weight loss and blood glucose reduction.

### SUMMARY

Aiming at the problems existing in the prior art, the present invention provides a *Bifidobacterium animalis* subsp*. lacti* (*B. animalis* subsp*. lacti*) strain, a food, a health product, a pharmaceutical composition and a probiotic preparation, and a preparation method therefore and use thereof.

In one aspect, the present invention provides a *B. animalis* subsp*. lacti* strain, wherein the *B. animalis* subsp*. lacti* strain has a deposit number of CGMCC No. 25680.

Optionally, the *B. animalis* subsp*. lacti* strain comprises a 16S rRNA gene represented by SEQ ID NO: 1.

Optionally, the *B. animalis* subsp*. lacti* strain has the ability to produce high levels of lactic acid and short-chain fatty acids.

The present invention also provides use of the *B. animalis* subsp*. lacti* strain for producing lactic acid and short-chain fatty acids.

Optionally, the short-chain fatty acid is selected from the group consisting of one or more of formic acid, acetic acid, propionic acid, butyric acid and isobutyric acid.

In addition, the *B. animalis* subsp*. lacti* strain of the present invention can inhibit various pathogenic bacteria, can tolerate artificial gastrointestinal fluids, can adhere to intestinal epithelial cells, and is sensitive to erythromycin, chloramphenicol, vancomycin, ampicillin, clindamycin, and streptomycin.

Optionally, the *B. animalis* subsp*. lacti* strain has antioxidant activity.

Further optionally, the *B. animalis* subsp*. lacti* strain has hydroxyl radical scavenging and DPPH radical scavenging activities, and has SOD enzyme activity.

The present invention also provides use of the *B. animalis* subsp*. lacti* strain in preparation of a composition for antioxidation.

Optionally, the antioxidantion is scavenging hydroxyl radicals and/or scavenging DPPH radicals.

Optionally, the *B. animalis* subsp*. lacti* strain has the ability to reduce oxidative stress.

The present invention also provides the of the *B. animalis* subsp*. lacti* strain in the preparation of a medicament for reducing oxidative stress.

In another aspect, the present invention provides a probiotic preparation for facilitating weight loss or assisting in lowering blood glucose, comprising the *B. animalis* subsp*. lacti* strain as described above and excipients.

Preferably, the probiotic preparation is a viable bacteria preparation, and the excipient is selected from the group consisting of one or more of starch, sucrose, trehalose and glycerol.

Preferably, the viable bacteria preparation comprises up to 5.0-7.0 × 10¹¹CFU/g of viable bacteria.

In another aspect, the present invention provides a method for preparing the probiotic preparation as described above, comprising the following steps: expanding the culturing of the *B. animalis* subsp*. lacti* strain as described above in an optimized liquid medium; collecting the bacteria; adding a protectant for resuspension, freeze-drying under vacuum, and pulverizing to obtain an active bacterial preparation.

In one embodiment, the *B. animalis* subsp*. lacti* strain of the present invention is derived from feces of healthy infants.

In another aspect, the present invention provides use of the *B. animalis* subsp*. lacti* strain, the viable bacteria preparation or the dead bacteria preparation thereof as described above in the preparation of a medicament for weight loss.

Optionally, the *B. animalis* subsp*. lacti* strain, the viable bacteria preparation or the dead bacteria preparation thereof is used to inhibit differentiation of 3T3-L1 preadipocytes.

Optionally, the *B. animalis* subsp*. lacti* strain, the viable bacteria preparation or the dead bacteria preparation thereof is used to reduce the triglyceride (TG) content in 3T3-L1 cells.

Optionally, the *B. animalis* subsp*. lacti* strain, the viable bacteria preparation or the dead bacteria preparation thereof is used to inhibit fat production.

Optionally, the *B. animalis* subsp*. lacti* strain, the viable bacteria preparation or the dead bacteria preparation thereof is used to treat metabolic diseases caused by obesity.

In yet another aspect, the present invention provides use of the *B. animalis* subsp*. lacti* strain, the viable bacteria preparation or the dead bacteria preparation thereof as described above in the preparation of a pharmaceutical composition for preventing or treating obesity and related diseases.

In yet another aspect, the present invention provides use of the *B. animalis* subsp*. lacti* strain, the viable bacteria preparation or the dead bacteria preparation thereof as described above in the preparation of a medicament for assisting in lowering blood glucose.

Optionally, the *B. animalis* subsp*. lacti* strain, the viable bacteria preparation or dead bacteria preparation thereof is used to inhibit the activity of dipeptidyl peptidase 4 (DPP-4) and α-glucosidase.

Optionally, the *B. animalis* subsp*. lacti* strain, the viable bacteria preparation or the dead bacteria preparation thereof is used to stimulate secretion of GLP-1 by NCI-H716 cells.

In yet another aspect, the present invention provides use of the *B. animalis* subsp*. lacti* strain, the viable bacteria preparation or the dead bacteria preparation thereof as described above in a pharmaceutical composition for preventing or treating diabetes.

In yet another aspect, the present invention provides a food or health product comprising the *B. animalis* subsp*. lacti* strain as described above.

In yet another aspect, the present invention provides a pharmaceutical composition comprising the *B. animalis* subsp*. lacti* strain as described above and a pharmaceutically acceptable excipient.

### Beneficial Effects

The 16S rRNA gene sequence of the *B. animalis* subsp*. lacti* strain of the present invention is different from other strains of the same species, and is unique. The strain and viable bacterial preparation or dead bacterial preparation thereof can inhibit the differentiation of 3T3-L1 preadipocytes, thereby achieving the effect of effectively inhibiting fat deposition, and having the function of weight loss. It can inhibit the activity of DPP-4 and α-glucosidase by producing higher levels of DPP-4 inhibitor and α-glucosidase inhibitor, and can stimulate NCI-H716 cells to secrete a high content of GLP-1, thereby having the effect of assisting in lowering blood glucose.

In addition, the strain also has the functions of producing high levels of lactic acid and short-chain fatty acids, inhibiting various pathogenic bacteria (with good antibacterial effects against *Escherichia coli*, *Salmonella*, *Staphylococcus aureus*, *Pseudomonas aeruginosa* and *Clostridium difficile*), and reducing oxidative stress.

The *B. animalis* subsp*. lacti* strain of the present invention can be used as an ordinary food or a health food, or as a drug to treat metabolic diseases caused by obesity, or to prevent or treat diabetes.

### BRIEF DESCRIPTION OF THE DRAWINGS

FIG. 1 shows a phylogenetic tree of *B. animalis* subsp*. lacti* HOM1119 strain.
FIG. 2 shows the RAPD cluster analysis diagram of *B. animalis* subsp*. lacti* strain HOM1119 strain of the present invention constructed based on the UPGMA method.
FIG. 3 shows the effect of *B. animalis* subsp*. lacti* HOM1119 strain of the present invention on the viability of 3T3-L1 preadipocytes.
FIG. 4 shows the Oil Red staining results of the *B. animalis* subsp*. lacti* HOM1119 strain of the present invention on the differentiation of 3T3-L1 preadipocytes.
FIG. 5 shows the effect of *B. animalis* subsp*. lacti* HOM1119 strain of the present invention on lipid accumulation of 3T3-L preadipocytes after differentiation. Note: * is p < 0.05 compared with the model control group, ** is p < 0.01 compared with the model control group, *** is p < 0.001 compared with the model control group.
FIG. 6 shows the effect of *B. animalis* subsp*. lacti* HOM1119 of the present invention on the triglyceride content of 3T3-L preadipocytes after differentiation. Note: * is p < 0.05 compared with the model control group, ** is p < 0.01 compared with the model control group, *** is p < 0.001 compared with the model control group.
FIG. 7 shows oil Red Staining results of the *B. animalis* subsp*. lacti* HOM1119 strain of the present invention on differentiation of 3T3-L1 preadipocytes.
FIG. 8 shows the effect of *B. animalis* subsp*. lacti* HOM1119 of the present invention on lipid accumulation of 3T3-L preadipocytes after differentiation. Note: * is p < 0.05 compared with the model control group, ** is p < 0.01 compared with the model control group, *** is p < 0.001 compared with the model control group.
FIG. 9 shows the effect of *B. animalis* subsp*. lacti* HOM1119 of the present invention on the triglyceride content of 3T3-L preadipocytes after differentiation. Note: * is p < 0.05 compared with the model control group, ** is p < 0.01 compared with the model control group, *** is p < 0.001 compared with the model control group.
FIG. 10 shows the effect of *B. animalis* subsp*. lacti* HOM1119 of the present invention on fasting blood glucose and glucose tolerance in a rat model with insulin-resistant and glucose/lipid metabolism disorder. Note: * is p < 0.05 compared with the model control group, ** is p < 0.01 compared with the model control group, *** is p < 0.001 compared with the model control group.

### Microbiological Collection

The *Bifidobacterium animalis* subsp*. lacti* (*B. animalis* subsp*. lacti*) HOM1119 strain of the present invention was deposited in the China General Microbiological Culture Collection Center (CGMCC) on September 9, 2022, with a Collection Center's address: Institute of Microbiology, Chinese Academy of Sciences, No. 3, Courtyard 1, Beichen West Road, Chaoyang District, Beijing; and a deposit number of CGMCC No. 25680.

The *B. animalis* subsp*. lacti* HOM1119 strain of the present invention was sent to the Institute of Microbiology, Chinese Academy of Sciences for identification in May 2023.

The detection and identification conclusions are as follows: under the conditions of this laboratory, according to the comprehensive analysis of experimental data such as cell morphology, physiological and biochemical characteristics, 16S rRNA gene sequence, and *tuf* gene sequence of the test strain, referring to the "*Bergey's Manual of Systematic Bacteriology*" and relevant research paper of International Journal of Systematic and Evolutionary Microbiology, the identification result of the test strain (strain number: HOM1119) is: *Bifidobacterium animalis* subsp*. lactis*.
The cell morphology of this strain is: polymorphic rod-shaped; the physiological and biochemical characteristics are: Gram-positive, catalase negative (-), oxidase negative (-); 16S rRNA gene sequence is shown in SEQ ID NO: 1, and *tuf* gene sequence is shown in SEQ ID NO: 9.

### DETAILED DESCRIPTION

The present invention discloses strains, characteristics and applications, and those skilled in the art can appropriately improve process parameters with reference to the content herein. It should be particularly noted that all similar substitutions and amendments are obvious to those skilled in the art, and they are considered to be included in the scope of the present invention. The method and application of the present invention have been described through the preferred embodiments. It is obvious that those skilled in the art can make amendments or appropriate changes and combinations to the method and application described herein without departing from the content, spirit and scope of the present invention to implement and apply the technology of the present invention.

In order to further illustrate the technical means and effects adopted by the present invention to achieve the intended purpose, the technical solution of the present invention will be further described below in conjunction with specific examples, but are not limited thereto. Any amendments or equivalent substitutions to the technical solutions of the present invention, without departing from the spirit and scope of the technical solutions of the present invention, shall fall within the protection scope of the present invention. The experimental methods without specific conditions in the following embodiments are implemented according to conventional methods and conditions in the art.

### Definition

As used herein, the term DPPH radical refers to 1,1-diphenyl-2-trinitrophenylhydrazine, also known as 1,1-diphenyl-2-picrylhydrazine (free radical); 1,2-dipicryl-2-trinitrophenylhydrazine; 1,2-diphenyl-2-picrylhydrazine. As a stable free radical, DPPH is stably in organic solvents, and its alcohol solution is purple, and needs to be stored in the dark at a low temperature. It has a single electron, so it can accept one electron or hydrogen ion, and has maximum absorption at a wavelength of 517 nm. In the presence of the free radical scavenger, the single electron of DPPH is captured to lighten its color, and the absorbance at the maximum light absorption wavelength decrease, and the degree of decrease is linear. The decrease in absorbance level indicated an increase in oxidation resistance, thereby evaluating the antioxidant capacity of the test sample. This antioxidation capacity is represented by the inhibition rate, and the higher the inhibition rate, the stronger the antioxidation capacity.

3T3-L1 preadipocytes (mouse embryonic fibroblasts) are normal cells of mice, also known as 3t3l1 preadipocytes. Mouse embryonic fibroblasts of 3T3-L1 adipose precursor cells are also known as 3t3l1 adipose precursor cells, which exhibit characteristics of contact inhibition of growth and can differentiate into adipocyte-like cells, and high serum culture medium can promote cell fat accumulation, etc.

3T3-L1 cell is a mouse embryonic fibroblast cell line, which is widely used in the study of metabolic diseases such as obesity and diabetes because it can differentiate into adipocyte-like cells in vitro. When cells progress from rapid division to confluence and contact inhibition, they undergo a reversal from preadipocyte to adipocyte-like cells. During culture, high serum content promotes fat accumulation.

It should be noted that the experimental methods used in the present invention are conventional methods unless otherwise specified.

Unless otherwise specified, the reagents and materials used in the present invention are all prepared by conventional methods or obtained commercially.

### Example

### Example 1. Isolation and Identification of B. animalis subsp. lacti HOM1119 strain

### (1) Preparation of Artificial Gastrointestinal Fluid

Artificial gastric fluid: Into 16.4 mL of dilute hydrochloric acid (1 mol/L), 800 mL of water was added, the pH was adjusted to 3.0, and 10g of pepsin was added. The mixture was shaken well, added with water to 1000 mL, centrifuged at 5000 rpm for 5 min, the supernatant was taken, filtered with a 0.22µm filter membrane for sterilization, and stored at -20°C for later use.

Artificial intestinal fluid: 6.8g of potassium dihydrogen phosphate was weighted, 500 mL of water was added, the pH was adjusted to 6.8 with a 0.4% (0.1 mol/L) sodium hydroxide solution. Separately, 10 g of pancreatin and 3 g of pig bile salt were weighted, an appropriate amount of water was added to dissolve them. The two solutions were mixed, added with water to 1000 mL, centrifuged at 5000 rpm for 5 min. The supernatant was taken, filtered with a 0.22 µm filter membrane for sterilization, and stored at -20°C for later use.

### (2) Preparation of Isolation Medium

MRS-Cys liquid medium: 0.5 g of L-cysteine hydrochloride was added to each liter of MRS broth medium (Cat. No. CM1163, OXOID, UK), sterilized at 121°C for 15 min, and stored at 2 ~ 8°C for one week in the dark.

MRS-Cys solid medium containing bromocresol purple: 0.04g of bromocresol purple was added to each liter of MRS solid medium (Cat. No.: CM1175, OXOID, UK), and stirred well. Sterilization was performed at 121°C for 15 min, about 15 mL of the medium was poured into each petri dish in a clean bench and was allowed to solidify for later use.

### (3) Isolation and Screening of B. animalis subsp. lactis HOM1119 strain

The *B. animalis* subsp*. lactis* of the present invention was isolated from feces of healthy infants.

The extracorporeal feces were collected with a sterile anaerobic tube and stored at low temperature under anaerobic environment. The experiment began on the day of sampling. About 1 g of fecal sample was weighted and added into an anaerobic tube containing 9 mL of MRS-Cys liquid medium, anaerobically incubated at 37°C for 24 h. After centrifugation at 8000 rpm for 10 min, the supernatant was discarded, and 10 mL of artificial gastric fluid was added. After mixing, the mixture was incubated anaerobically at 37°C for 3 h, centrifuged at 8000 rpm for 10 min, the supernatant was discarded. 10 mL of artificial intestinal fluid was added, after mixing, the mixture was incubated anaerobically at 37°C for 3 h. The sample was diluted by a 10-fold dilution method to a concentration of 10⁻⁶. 100 µL of each dilution was taken from the original solution to the concentration of 10⁻⁶, and spread onto MRS-Cys solid medium plates containing bromocresol purple, statically incubated under anaerobic conditions at 37°C for 72 h.

Single colonies with yellowing edges were picked, streaked, and purified 3-4 times until single colonies were observed. Gram staining and microscopy of colony morphology were performed simultaneously. Single colonies were then transferred to liquid medium for pure culture, preserved with glycerol, and stored at -80°C.

### (4) Morphology Observation of Bifidobacterium animalis subsp. lactis HOM1119 strain

*B. animalis* subsp*. lactis* HOM1119 strain was anaerobically cultured on MRS agar medium at 37°C for 48 h. The colonies were round, milky white, the surface was moist and smooth, the center was raised, and the edges were neat. The diameter of the colonies was about 2.5 mm. It was observed under an optical microscope that the bacteria were rod-shaped with enlarged ends, club-shaped or Y-shaped bacteria. The size of the bacteria was about (0.5 ~ 0.9) µm × (3 ~ 6) µm, arranged individually or in pairs, Gram-positive, and did not form spores. At the same time, the other two strains with the same morphology were screened, named *B. animalis* subsp*. lactis* BH13-25 and BH13-33 strains.

### (5) Identification of B. animalis subsp. lactis HOM1119 Strain

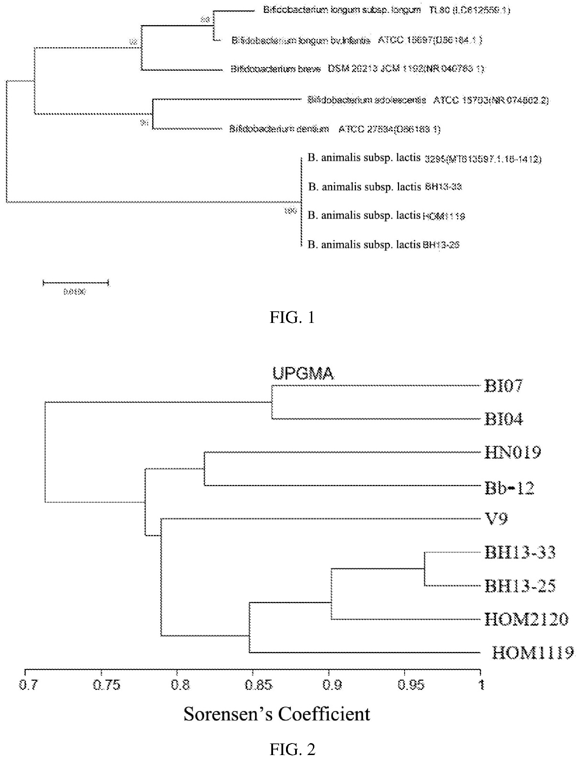

16S rDNA identification of the strain: DNA was extracted from three stored strains HOM1119, BH13-25 and BH13-33, respectively. 16S rDNA amplification was performed. PCR amplification was performed using universal primers 27F: SEQ ID NO: 7; 1492 R: SEQ ID NO: 8(see Table 1) and agarose gel electrophoresis was performed. Then, the gel was cut and recovered, sequenced, and the isolated strains were subjected to 16S rDNA sequencing. According to its 16S rDNA sequence, it was aligned in the NCBI database using the BLAST tool, and the phylogenetic tree was constructed using Mega 7.0 software. As shown in FIG. 1, the identification results of the three strains are all *B. animalis* subsp*. lactis*, which were respectively named *B. animalis* subsp*. lactis* HOM1119, BH13-25 and BH13-33 strains, wherein the sequence of the 16S rRNA gene of the *B. animalis* subsp*. lactis* HOM1119 strain was shown in SEQ ID NO: 1:

CTTACACATGCAGTCGAACGGATCCCTGGCAGCTTGCTGTCGGGGTGA
GAGTGGCGAACGGGTGAGTAATGCGTGACCAACCTGCCCTGTGCACCGGAATAGCTC
CTGGAAACGGGTGGTAATACCGGATGCTCCGCTCCATCGCATGGTGGGGTGGGAAAT
GCTTTTGCGGCATGGGATGGGGTCGCGTCCTATCAGCTTGTTGGCGGGGTGATGGCCC
ACCAAGGCGTTGACGGGTAGCCGGCCTGAGAGGGTGACCGGCCACATTGGGACTGA
GATACGGCCCAGACTCCTACGGGAGGCAGCAGTGGGGAATATTGCACAATGGGCGCA
AGCCTGATGCAGCGACGCCGCGTGCGGGATGGAGGCCTTCGGGTTGTAAACCGCTTT
TGTTCAAGGGCAAGGCACGGTTTCGGCCGTGTTGAGTGGATTGTTCGAATAAGCACC
GGCTAACTACGTGCCAGCAGCCGCGGTAATACGTAGGGTGCGAGCGTTATCCGGATTT
ATTGGGCGTAAAGGGCTCGTAGGCGGTTCGTCGCGTCCGGTGTGAAAGTCCATCGCC
TAACGGTGGATCTGCGCCGGGTACGGGCGGGCTGGAGTGCGGTAGGGGAGACTGGA
ATTCCCGGTGTAACGGTGGAATGTGTAGATATCGGGAAGAACACCAATGGCGAAGGC

AGGTCTCTGGGCCGTCACTGACGCTGAGGAGCGAAAGCGTGGGGAGCGAACAGGAT
TAGATACCCTGGTAGTCCACGCCGTAAACGGTGGATGCTGGATGTGGGGCCCTTTCCA
CGGGTCCCGTGTCGGAGCCAACGCGTTAAGCATCCCGCCTGGGGAGTACGGCCGCAA
GGCTAAAACTCAAAGAAATTGACGGGGGCCCGCACAAGCGGCGGAGCATGCGGATT
AATTCGATGCAACGCGAAGAACCTTACCTGGGCTTGACATGTGCCGGATCGCCGTGG
AGACACGGTTTCCCTTCGGGGCCGGTTCACAGGTGGTGCATGGTCGTCGTCAGCTCG
TGTCGTGAGATGTTGGGTTAAGTCCCGCAACGAGCGCAACCCTCGCCGCATGTTGCC
AGCGGGTGATGCCGGGAACTCATGTGGGACCGCCGGGGTCAACTCGGAGGAAGGTG
GGGATGACGTCAGATCATCATGCCCCTTACGTCCAGGGCTTCACGCATGCTACAATGG
CCGGTACAACGCGGTGCGACACGGTGACGTGGGGCGGATCGCTGAAAACCGGTCTC
AGTTCGGATCGCAGTCTGCAACTCGACTGCGTGAAGGCGGAGTCGCTAGTAATCGCG
GATCAGCAACGCCGCGGTGAATGCGTTCCCGGGCCTTGTACACACCGCCCGTCAAGT
CATGAAAGTGGGTAGCACCCGAAGCCGGTGGCCCGACCCTTGTGGGGGGAGCCGTC
TAA

In addition, *B. animalis* subsp*. lactis* BH13-25 and BH13-33 strains isolated simultaneously with *B. animalis* subsp*. lactis* HOM1119 strain of the present invention are used as control strains in the present invention, and the sequences of 16S rRNA genes thereof were shown in SEQ ID NOs: 10 and 11, respectively. By comparing the 16S rRNA gene sequences of *B. animalis* subsp*. lactis* HOM1119 strain of the present invention and *B. animalis* subsp*. lactis* BH13-25 and BH13-33 strains used as control strains in the present invention, it can be seen that strain BH13-25 has 1401 identical bases to strain HOM1119 of the present invention, with a sequence similarity of 98.87%; strain BH13-33 has 1396 identical bases to strain HOM1119 of the present invention, with a sequence similarity of 98.52%.

(6) Identification of strain by random amplification polymorphism DNA(RAPD): DNA was extracted from the stored strain, using the DNA of the strain as a template, and 5 primers OPA-02: SEQ ID NO: 2; OPA-18: SEQ ID NO: 3; OPL-07: SEQ ID NO: 4; OPL-16: SEQ ID NO: 5; OPM-05: SEQ ID NO: 6(see Table 1) were respectively used to amplify DNA fragments capable of showing polymorphism by PCR, which showed different DNA differences after gel electrophoresis, and the results were analyzed by cluster analysis software, as shown in FIG. 2. As shown in FIG. 2, *B. animalis* subsp*. lactis* HOM1119 strain was different from some commercial *B. animalis* subsp*. Lactis* strain, as well as two other strains, BH13-25 and BH13-33, which were isolated at the same time of this invention, and was unique.

**Table 1**

| Primers | Sequence | SEQ ID NO |
|---|---|---|
| OPA-02 | 5'- TGCCGAGCTG -3' | 2 |
| OPA-18 | 5'- AGGTGACCGT -3' | 3 |
| OPL-07 | 5'- AGGCGGGAAC -3' | 4 |
| OPL-16 | 5'- AGGTTGCAGG -3' | 5 |
| OPM-05 | 5'- GGGAACGTGT -3' | 6 |
| 27F | 5'- AGTTTGATCMTGGCTCAG -3' | 7 |
| 1492R | 5'- GGTTACCTTGTTACGACTT -3' | 8 |

### Example 2. Test on the Ability to Inhibit Common Pathogenic Bacteria

### (1) Indicator Bacteria Activation

The indicator strains *Escherichia coli* ATCC8739; *Staphylococcus aureus* ATCC6538; *Salmonella typhimurium* ATCC14028; *Pseudomonas aeruginosa* ATCC9027; *Listeria monocytogenes* ATCC19111 and *Clostridium difficile* ATCC9689 were all purchased from the China Center of Industrial Culture Collection. Indicator strains (*Escherichia coli* ATCC8739; *Staphylococcus aureus* ATCC6538; *Salmonella typhimurium* ATCC14028; *Pseudomonas aeruginosa* ATCC9027; *Listeria monocytogenes* ATCC19111) were inoculated into TSB medium (tryptone soybean broth medium, where T is tryptone, S is soybean, and B is broth) at an inoculation amount of 1% of the total medium, and cultured aerobically at 37°C and 180 rpm for 24h for later use. *C. difficile* ATCC9689 was inoculated into Brain Heart Infusion Broth (BHI) medium at an inoculation amount of 1% of the total amount of the medium, and cultured anaerobically at 37°C for 24 h for later use.

### (2) Activation of B. animalis subsp. lactis strain

The *B. animalis* subsp*. lactis* HOM1119 strain, cryopreserved at -80°C as prepared in Example 1, and the control strain were respectively inoculated into the sterilized MRS-Cys liquid medium described in Example 1 at an inoculation amount of 1% of the total amount of the medium. After being statically cultured under anaerobic conditions at 37°C for 24 hours and activated twice, the strain fermentation solution was obtained. Then, the strain fermentation solution was centrifuged at 8000 rpm for 10 min, and the supernatant was taken for bacteriostatic test.

### (3) Plate Preparation

The sterilized TSA medium was heated to completely melt, poured into a petri dish, placed on a horizontal platform to form an agar layer with a uniform thickness, and solidified. The indicator strains were added to the TSA medium, shook evenly, poured into a pre-prepared TSA blank agar plate, and allowed to stand for coagulation.

### (4) Bacteriostatic Test

The Oxford cup was gently placed on the plate with sterile forceps, with a certain distance between the holes, and 0.2 mL of the supernatant of the lactic acid bacteria fermentation solution to be tested was respectively added thereto. After the mixture was placed in a refrigerator at 4°C for 24 hours, cultured in an incubator at 37°C for at least 18 hours to observe the occurrence of the inhibition zone. The inhibition zone was formed and then measured with a ruler. The liquid medium (MRS-Cys) in Example 1 was used as a negative control. Each sample was tested in triplicate. The bacteriostatic results were shown in Table 2.

**Table 2. Inhibitory Effect of B. animalis subsp. lactis HOM1119 on Pathogenic Bacteria**

| Sample | *E. coli* | *Salmonel la* | *Staphylococc us aureus* | *Pseudomonas aeruginosa* | *Listeria monocytogenes* | *Clostridium difficile* |
|---|---|---|---|---|---|---|
| MRS-Cys (Negative Control) | - | - | - | - | - | - |
| BH13-25 | + | + | - | - | + | - |
| BH13-33 | + | + | - | - | + | - |
| HOM1119 | +++ | ++ | ++ | ++ | + | ++ |

| | | | | | | |
|---|---|---|---|---|---|---|
| Note: "-" no bacteriostatic activity; "+" 11-16 mm; "++" 17-22 mm; "+++" ≥ 23 mm | | | | | | |

It can be seen from Table 2 that *B. animalis* subsp*. lactis* HOM1119 has an inhibitory effect on all 6 pathogenic bacteria. Compared with the other 2 strains of *B. animalis* subsp*. lactis* strain, the HOM1119 has a better inhibitory effect on *E. coli, Salmonella*, *Staphylococcus aureus*, *Pseudomonas aeruginosa* and *Clostridium difficile*, indicating that it has a better ability to inhibit pathogenic bacteria.

### Example 3. Test on the Ability to Pass Through Gastrointestinal tract

### (1) Activation of Strains

The *B. animalis* subsp*. lactis* HOM1119 strain, cryopreserved at -80°C as prepared in Example 1, and the control strains BH13-25 and BH13-33 were respectively inoculated into the sterilized MRS-Cys liquid medium described in Example 1 at an inoculation amount of 1% of the total amount of the medium, statically cultured under anaerobic conditions at 37°C for 24 hours. After being activated twice, the strain fermentation solution was obtained.

### (2) Preparation of Artificial Gastric Fluid

Into 16.4 ml of dilute hydrochloric acid, about 800 ml of water and 10g of pepsin were added, shaken well, the pH was adjusted to 3.0, the water was added to 1000 ml, and filtered with a 0.2µm microporous membrane for later use.

### (3) Preparation of Artificial Intestinal Fluid

6.8g of potassium dihydrogen phosphate was weighted and dissolved in 500 mL of water, and the pH was adjusted to 6.8. Separately, 10 g of trypsin and 3 g of pig bile salt were weighted, dissolved in an appropriate amount of water. The two solutions were mixed, water was added to 1000 mL, and filtered with a 0.22 um sterile membrane under a sterile environment for later use.

### (4) Evaluation of Survival Capacity of Strains in Simulated Gastrointestinal tract

1 mL of activated bacterial solution of the strain to be tested was added to 9 mL of artificial gastric fluid (pH3.0), mixed well, and the number of viable bacteria was counted, and incubated at 37°C for 3 h in an incubator before counting the viable cells again. After culturing in artificial gastric fluid for 3 h, all the bacterial cells were transferred into an equal volume of artificial intestinal fluid (pH6.8), mixed well, and incubated at 37°C. The viable cell counts were performed on MRS medium at 3 h and 24 h respectively, and the survival rate was calculated by the following formula:
$Survival rate in the gastric fluid for 3 hours \left(%\right) = \left[logCFUN1/logCFUN0\right] \times 100 %$
$Survival rate in the intestinal fluid for 3 hours \left(%\right) = \left[logCFUN2/logCFUN0\right] \times 100 %$ 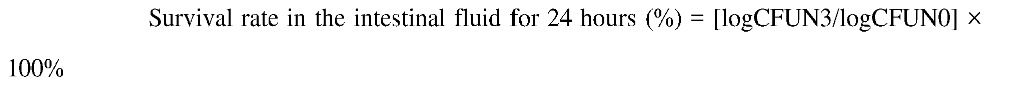 Survival rate in the intestinal fluid for 24 hours (%) = [logCFUN3/logCFUN0] × 100%

N0= the viable count of *B. animalis* subsp*. lactis* strain before treatment, N1= the viable count of *B. animalis* subsp*. lactis* strain after 3 hours of treatment with gastric fluid, N2= the viable count of *B. animalis* subsp*. lactis* strain after 3 hours of treatment with intestinal fluid, N3= the viable count of *B. animalis* subsp*. lactis* strain after 24 hours of treatment with intestinal fluid. Data were analyzed using SPSS 25.0 software via one-way ANOVA and Duncan's multiple comparisons. The results are shown in Table 3.

**Table 3. Survival rate of B. animalis subsp. lactis HOM1119 in the simulated gastrointestinal fluids**

| Strain Name | Survival rate in gastric fluid for 3 hours | Survival rate in intestinal fluid for 3 hours | Survival rate in intestinal fluid for 24 hours |
|---|---|---|---|
| BH13-33 | 100.27±0.92% | 46.33±4.73%^{C} | 33.29±2.59%^{C} |
| BH13-25 | 101.04±1.01% | 65.86±1.15%^{B} | 42.06±1.99%^{B} |
| HOM1119 | 98.51±1.07% | 97.85±0.17%^{A} | 85.33±0.52%^{A} |

| | | | |
|---|---|---|---|
| Note: In the same column, different capital letters for the superscript indicate highly significant differences (p < 0.01). | | | |

It can be seen from Table 3 that after 3 hours of treatment with simulated gastric fluid, the survival rate of *B. animalis* subsp*. lactis* HOM1119 was higher than 98%. After further treatment with simulated intestinal fluid for 24 hours, the survival rate can still reach up to 85% or more, and was very significantly (p < 0.01) superior to the tolerance to intestinal fluid of the other two strains of *B. animalis* subsp*. lactis* BH13-25 and BH13-33. It indicated that *B. animalis* subsp*. lactis* HOM1119 strain had a high survival rate in the intestinal tract, laying a foundation for its colonization and effective function in the intestinal tract.

### Example 4. Test on Ability to Adhere to Intestinal Epithelial Cells

### (1) Preparation of Cell Medium

Complete medium: Into high-sugar DMEM medium, 10% of inactivated fetal bovine serum (FBS) and 1% (v/v) of antibiotic (100 U/mL penicillin, 100 µg/mL streptomycin) were added, mixed and then stored at 4 °C.

Incomplete medium: Into high-sugar DMEM medium, 10% of inactivated fetal bovine serum (FBS) was added, mixed and then stored at 4 °C.

### (2) Cell Recovery and Culture

Human colorectal adenocarcinoma Caco-2 cells were purchased from the Cell Resource Center, Institute of Basic Medical Sciences, Chinese Academy of Medical Sciences. The cells were resuspended in a fresh culture solution, uniformly dispersed in a culture flask, cultured at 37°C under a gas condition of 5% CO₂ and 95% air. The culture solution was replaced every 48 h during recovery. When the cells grew well (80% confluence), Caco-2 cells were digested with trypsin-EDTA solution at 37°C. The cell concentration was adjusted to 2 × 10⁵ cells/mL after digestion, and the cells were inoculated in a 24-well plate and cultured until the cells reached 80% confluence.

### (3) Activation of B. animalis subsp. lactis strain

The *B. animalis* subsp*. lactis* HOM1119 strain cryopreserved at -80°C as prepared in Example 1, was inoculated into the sterilized MRS-Cys liquid medium described in Example 1 at an inoculation amount of 1% of the total amount of the medium, statically cultured under anaerobic conditions at 37°C for 24 hours and activated twice, and then the strain fermentation broth was obtained.

### (4) Adhesion Experiment

The bacterial cells grown in the corresponding medium were collected by centrifugation at 8000 rpm for 10 min. After washing the bacterial cells with DPBS for 3 times, the bacterial cells were resuspended in the incomplete medium, and the concentration of the bacterial cells was adjusted to 10⁸ cfu/mL. 1 mL of the above bacterial suspension was added to a 24-well plate containing Caco-2 cells that have grown to a single layer, and incubated at 37°C for 2 h in a 5% CO₂ incubator. After incubation, the cells were washed with sterile DPBS for 3 times. The Caco-2 cells were digested with trypsin-EDTA solution at a temperature of 37°C, the number of cells and the number of viable bacteria before and after adhesion were counted. Each sample was tested in triplicate. Data were analyzed using SPSS 25.0 software via one-way ANOVA and Duncan's multiple comparisons. The results were shown in Table 4.

**Table 4. Adhesion Capacity of B. animalis subsp. lactis HOM1119 strain to Caco-2 cells**

| Strain Name | Adhesion Rate (%) | Adhesion Index (CFU/Cell) |
|---|---|---|
| BH13-33 | 1.97±0.73%^{B} | 1.82±0.77^{b} |
| BH13-25 | 2.44±0.56%^{B} | 2.48±0.31^{ab} |
| HOM1119 | 5.14±0.41%^{A} | 4.56±0.20^{A} |

| | | |
|---|---|---|
| Note: In the same column, different capital letters for the superscript indicate highly significant differences (p < 0.01). | | |

The results in Table 4 showed that the adhesion rate of *B. animalis* subsp*. lacti* HOM1119 strain to human colon cancer cells Caco-2 was 5.14%, and an adhesion index was 4.56, which was better than the other two strains of *B. animalis* subsp*. lacti* strain, and has a better capacity to adhere to intestinal epithelial cells.
$Adhesion index = number of bacteria after adhesion / number of cells per plate$ 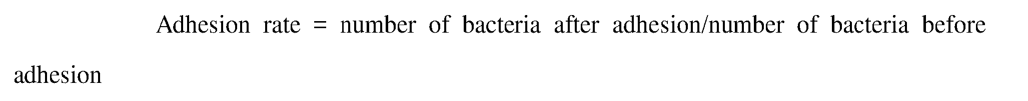 Adhesion rate = number of bacteria after adhesion/number of bacteria before adhesion

### Example 5. Test on the Ability to Produce Lactic Acid and Short-Chain Fatty Acid

The *B. animalis* subsp*. lactis* HOM1119 strain cryopreserved at -80°C as prepared in Example 1, was inoculated into the sterilized MRS-Cys liquid medium described in Example 1 at an inoculation amount of 1% of the total amount of the medium, statically cultured under anaerobic conditions at 37°C for 24 hours. After being activated twice, the strain fermentation solution was obtained. Then the strain fermentation solution was centrifuged at 8000 rpm for 10 min, and the supernatant was taken and the contents of lactic acid and short-chain fatty acids were determined by gas chromatography. The MRS-Cys liquid medium described in Example 1 was used as a negative control, the other two strains of *B. animalis* subsp*. lactis* strain were used as positive controls. Each sample was performed in triplicate. The data were analyzed by SPSS 25.0 software for one-way ANOVA analysis and Duncan's multiple comparison. The results were shown in Table 5.

**Table 5. Lactic acid and short-chain fatty acid production capacity of B. animalis subsp. lactis HOM1119**

| Sample | Lactic Acid (g/L) | Acetic Acid (g/L) | Formic Acid (g/L) | Propionic Acid (mg/L) | Butyric Acid (mg/L) | Isobutyric Acid (mg/L) |
|---|---|---|---|---|---|---|
| MRS-Cys (Negative Control) | 0.54±0.01^{C} | 2.13±0.01^{C} | 0.16±0.00^{B} | 11.4±0.2^{B} | 12.3±0.3^{D} | 10.0±0.2^{C} |
| BH13-25 | 0.93±0.04^{B} | 4.07±0.10^{B} | 0.19±0.01^{B} | 19.7±0.1^{B} | 14.4±0.1^{C} | 10.4±0.1^{BC} |
| BH13-33 | 0.92±0.01^{B} | 4.04±0.03^{B} | 0.17±0.01^{B} | 20.3±0.4^{B} | 15.2±0.2^{B} | 10.8±0.1^{B} |
| HOM1119 | 1.36±0.03^{A} | 4.47±0.21^{A} | 1.36±0.02^{A} | 21.7±0.1^{A} | 16.0±0.1^{A} | 11.8±0.0^{A} |

| | | | | | | |
|---|---|---|---|---|---|---|
| Note: In the same column, different capital letters for the superscript indicate highly significant differences (p < 0.01). | | | | | | |

It can be seen from Table 5 that that compared with other strains of *B. animalis* subsp*. lactis*, *B. animalis* subsp*. lactis* HOM2120 strain had a stronger ability to produce lactic acid and short-chain fatty acids (acetic acid, formic acid, propionic acid, butyric acid and isobutyric acid).

### Example 6. Test on Antibiotic Susceptibility

The drug susceptibility test was carried out according to ISO10932-2010, "Milk and milk products - Determination of the minimal inhibitory concentration (MIC) of antibiotics applicable to bifidobacteria and non-enterococcal lactic acid bacteria (LAB)", established by the International Organization for Standardization.

### (1) Preparation of Medium

MRS-Cys liquid medium: preparation method is the same as in Example 1.

LSM-Cys liquid medium: 21.06g of Iso-Sensitest medium (Cat. No. CM0473B, OXOID, UK) was weighed, 5.2 g of MRS Broth and 0.3 g of L-cysteine hydrochloride were weighed, added with water to 0.5 L, the pH was adjusted to 6.85 ± 0.1, and sterilized at 121°C for 15 min, and the pH should be 6.7 ± 0.1 for later use, and stored at 2 - 8°C for one week in the dark.

### (2) Activation and Proliferation of B. animalis subsp. lactis

The *B. animalis* subsp*. lactis* HOM1119 strain cryopreserved at -80°C as prepared in Example 1, was inoculated into the sterilized liquid medium (MRS-Cys) described in Example 1 at an inoculation amount of 1% of the total amount of the medium, statically cultured under anaerobic conditions at 37°C for 24 hours. After being activated twice, the strain fermentation solution was obtained. The activated *B. animalis* subsp*. lactis* HOM1119 strain was propagated on MRS-Cys agar medium, and statically cultured under anaerobic conditions at 37°C for 48 hours.

### (3) Preparation of Antibiotic Microdilution Plate

0.0512g of antibiotic was weighed, into which 10 mL of solvent was added, wherein chloramphenicol and erythromycin were dissolved in ethanol (without filtration), ampicillin was dissolved in phosphate buffer (pH 8.0, 0.1 mol/L), and other antibiotics were dissolved in water. After shaking to dissolve, filtered with a 0.22µm filter membrane, sub-packaged into EP tubes, with a concentration of 5120 µg/mL (5.12 mg/mL), and stored at -20°C for later use. The antibiotic stock solution was diluted with water (ampicillin with phosphate buffer) to a suitable concentration range. 50µL of diluent was added to the wells of the microdilution plate.

### (4) Preparation of Bacterial Suspensions

Individual colonies were picked from agar plates. The obtained colonies were suspended in sterile culture tubes containing 2 mL to 5 mL of sterile saline. The obtained colonies were suspended in pre-reduced LSM-Cys liquid medium. Colonies were suspended until the solution turbidity reached McFarland Standard 1 or the optical density at 625 nm was 0.16 - 0.2 using the spectrophotometer. The suspension was approximately equivalent to 3 × 10⁸ CFU/mL. The bacterial suspension was diluted with the recommended medium and the bacterial suspension was diluted 500-fold with MRS-Cys liquid medium, as the antibiotic solution will dilute the medium by two times. The diluted bacterial suspension was dispensed within 30 minutes after the preparation. When the freeze plate was used, the frozen antibiotic solution was thawed under anaerobic conditions immediately prior to use. 50µL of the diluted suspension was dispensed into each well of the microdilution plate (about 3 × 10⁴ CFU/well). The plates were statically incubated at 37°C under anaerobic conditions for 48 h. When an anaerobic jar was used, the plates were capped between each plate to create a homogeneous environment in the anaerobic jar. Each experiment was repeated three times, and both positive control group and a negative control group were set. The positive control wells did not contain antibiotics, but included the test strains and medium containing solvents to dissolve the highest concentrations of antibiotics. Negative control wells did not contain the test strain and antibiotics, but included medium.

### (5) Reading MIC Results

After 48 hours of incubation, the MIC was visually read. After incubation, the negative control wells were checked for visible bacterial growth. If contamination was found, all data generated by the relevant strain was rejected. Note: If there was no growth in the positive control well, it indicated that the tested strain was sensitive to the solvent used to dissolve the antibiotic. In this case, reading the MIC for that particular antibiotic was meaningless. If the negative and positive controls were normal, the growth of bacteria in each antibiotic was determined visually by comparing them with the positive control. Preferably, the microdilution plate was placed on the top of the frame of the magnifying glass, and the table lamp providing indirect light for easy reading. The growth of bacteria was easily detected under the magnifying glass as sediment at the bottom of the well. Any series of wells in which growth discontinuities were observed were discarded (e.g., grown at 16 µg/mL and 64 µg/mL, but not at 32µg/mL). The endpoint was defined as the lowest antibiotic concentration at which no growth was visually observed. The concentration was the MIC of that antibiotic for that specific strain. Each sample was tested in triplicate. *Lactobacillus plantarum* ATCC14917, *B. animalis* subsp*. lactis* BH13-25 and BH13-33 strain were used as positive control strains. The antibiotic susceptibility results of *B. animalis* subsp*. lactis* HOM1119 were shown in Table 6. The MIC results of *L. plantarum* ATCC14917 were consistent with those shown in the Appendix of ISO10932-2010, indicating that the experimental method was accurate (*L. plantarum* is a control strain recommended by the MIC method, and this strain was used to demonstrate the accuracy of the method). According to the antibiotic resistance standards for bacterial strains used in food established by the European Food Safety Authority (EFSA) in 2012, the antibiotic resistance standard for using strains in food was formulated, and it can be seen that *B. animalis* subsp*. lactis* HOM1119 strain was sensitive to erythromycin, chloramphenicol, vancomycin, ampicillin, clindamycin and streptomycin. The results were consistent with *B. animalis* subsp*. lactis* BH13-25 and BH13-33 strains. Therefore, the *B. animalis* subsp*. lactis* HOM1119 strain was relatively safe.

**Table 6. Antibiotic susceptibility results of B. animalis subsp. lactis HOM1119**

| Antibiotics | Judgment Criteria | | *B. animalis* subsp*. lactis* HOM1119 | | *B. animalis* subsp*. lactis* BH13-25 (Control Strain) | | *B. animalis* subsp*. lacti* BH13-33 (Control Strain) | | *L. plantarum* ATCC14917 (Control Strain) | |
|---|---|---|---|---|---|---|---|---|---|---|
| | Sensitivi ty (S) | Drug Resistance (R) | MIC Value | Results | MIC Value | Results | MIC Value | Results | MIC Value | Results |
| Erythromycin | ≤1 | >1 | 0.5±0 | S | 0.5±0 | S | 0.5±0 | S | 1±0 | S |
| Chloramphenicol | ≤4 | >4 | 2±0 | S | 1.6±0.5 | S | 1±0 | S | 0.83±0.29 | S |
| Tetracycline | ≤8 | >8 | 16±0 | R | 16±0 | R | 16±0 | R | 8±0 | S |
| Vancomycin | ≤2 | >2 | 0.5±0 | S | 0.5±0 | S | 0.5±0 | S | 128±0 | / |
| Ampicillin | ≤2 | >2 | 0.2±0 | S | 0.2±0 | S | 0.3±0.1 | S | 0.5±0 | S |
| Clindamycin | ≤1 | >1 | 0.063±0 | S | 0.063±0 | S | 0.063±0 | S | 0.5±0 | S |
| Gentamicin | ≤64 | >64 | 128±0 | R | 128±0 | R | 128±0 | R | 4±0 | S |
| Streptomycin | ≤128 | >128 | 64±0 | S | 64±0 | S | 64±0 | S | 128±0 | / |

### Example 7. Test on Antioxidant Activity

### (1) Activation of Strains

A commercial strain *Lactobacillus rhamnosus* GG(ATCC53103) strain (purchased from China Center of Industrial Culture Collection) (GG is a commercial strain, which is a strain with antioxidant effect in the literature) and *B. animalis* subsp*. lactis* BH13-25 and BH13-33 strains were selected as control strains. The *B. animalis* subsp*. lactis* HOM1119, BH13-25 and BH13-33 strains and *L. rhamnosus* GG strain were inoculated into the sterilized MRS-Cys liquid medium described in Example 1 at an inoculation amount of 1% of the total amount of the medium, statically cultured under anaerobic conditions at 37°C for 24 hours and activated twice, and then the strain fermentation solution was obtained. Then the strain fermentation solution was centrifuged at 8000 rpm for 10 min, and the strain concentration was adjusted to 1× 10¹⁰ CFU/mL with a sterile 0.9% sodium chloride solution for testing.

### (2) Determination of Antioxidant Activity

The oxidation capacity was determined according to the instructions of the Total Antioxidant Capacity (T-AOC) Assay Kit, the Total Superoxide Dismutase (SOD) Assay Kit, the DPPH Free Radical Scavenging Capacity Kit and the Hydroxyl Free Radical Assay Kit produced by Nanjing Jiancheng Biotechnology Research Institute. Each treatment was performed in triplicate. Data were analyzed using SPSS 25.0 software for one-way ANOVA and Duncan's multiple comparisons. The results were shown in Table 7.

**Table 7. In vitro antioxidant activity results of B. animalis subsp. lacti HOM1119 strain ( x ± SD)**

| Strain Name | T-AOC (U/mL) | OH Scavenging Rate (%) | DPPH Scavenging Rate (%) | SOD(U/mL) |
|---|---|---|---|---|
| HOM1119 | 9.91±0.40^{A} | 74.11% ±2.12%^{A} | 93.13%±1.03%^{A} | 28.48±0.44^{A} |
| GG | 10.24±0.25^{A} | 64.84%±1.36%^{B} | 85.62%±0.34%^{AB} | 27.42±0.56^{AB} |
| HOM13-33 | 6.58±0.19^{B} | 60.75%±1.95%^{B} | 79.62%±6.10%^{BC} | 26.04%±0.40%^{B} |
| HOM13-25 | 6.54±0.25^{B} | 59.01%±3.24%^{B} | 74.18%±5.08%^{C} | 21.58%±0.87%^{C} |

| | | | | |
|---|---|---|---|---|
| Note: In the same column, different capital letters for the superscript indicate highly significant differences (p < 0.01). | | | | |

It can be seen from Table 7 that, compared with the control strain, the *B. animalis* subsp*. lactis* HOM1119 strain exhibited significantly higher hydroxyl radical scavenging capacity, DPPH radical scavenging capacity, and SOD enzyme activity (p < 0.01) than that of *L. rhamnosus* GG. Its total antioxidant capacity (T-AOC), hydroxyl radical scavenging capacity, DPPH radical scavenging capacity, and SOD enzyme activity were all significantly higher than those of *B. animalis* subsp. *lactis* BH13-25 and BH13-33 strain. It indicated that the *B. animalis* subsp. *lacti* HOM1119 strain has the effect of alleviating the damage caused by excessive reactive oxygen species in the body, thereby protecting P cells producing insulin.

### Example 8. DPP- 4 Inhibitory and α-Glucosidase Inhibitory Tests

### (1) Activation of Strains

A commercial strain *L. rhamnosus* GG(ATCC53103) strain (purchased from China Center of Industrial Culture Collection) (GG is a commercial strain, which is a strain with antioxidant effect in the literature) and *B. animalis* subsp. *lactis* BH13-25 and BH13-33 strains were selected as control strains. The *B. animalis* subsp. *lactis* HOM1119, BH13-25 and BH13-33 strains and *L. rhamnosus* GG strain were inoculated into the sterilized MRS-Cys liquid medium described in Example 1 at an inoculation amount of 1% of the total amount of the medium, statically cultured under anaerobic conditions at 37°C for 24 hours and activated twice, and then the strain fermentation solution was obtained. Then the strain fermentation solution was centrifuged at 8000 rpm for 10 min, and the strain concentration was adjusted to 1× 10¹⁰ CFU/mL with a sterile 0.9% sodium chloride solution. The strain fermentation solution was anaerobically cultured for 8 h, centrifuged at 8000 rpm for 10 min, and the supernatant was collected for later use.

### (2) DPP-4 Inhibitory and α-Glucosidase Inhibitory Assay

In vitro screening of hypoglycemic function was performed using a DPP-4 inhibitor screening kit (Abnova#KA1311) and an α-glucosidase inhibitor screening kit (Biovision#K938). The inhibition rates of the strain samples against the above two enzymes were calculated, with three replicates for each treatment. The data were analyzed using SPSS 25.0 software for one-way ANOVA and Duncan's multiple comparisons. The results were shown in Table 8.

**Table 8. Results of Inhibition of DPP- 4 and Inhibition of α-Glucosidase by B. animalis subsp. lacti HOM1119 strain ( x ± SD)**

| Strain Name | DPP- 4 inhibition rate (%) | α-Glucosidase Inhibition Rate (%) |
|---|---|---|
| HOM1119 | 22.45%±1.37%^{A} | 20.40%±0.72%^{A} |
| GG | 12.33%±1.27%^{B} | 6.60%±0.56%^{B} |
| HOM13-33 | 7.80%±0.37%^{C} | 4.83%±0.87%^{B} |
| HOM13-25 | 5.43%±0.51%^{C} | 4.64%±0.5%^{C} |

| | | |
|---|---|---|
| Note: In the same column, different capital letters for the superscript indicate highly significant differences (p < 0.01). | | |

It can be seen from Table 8 that, compared with the control strains, *B. animalis* subsp*. lactis* BH13-25, BH13-33 and *L. rhamnosus* GG, the *B. animalis* subsp*. lactis* HOM1119 strain exhibited significantly higher inhibition rate against DPP-4 and α-glucosidase, and the difference was extremely significant (p < 0.01). It indicated that *B. animalis* subsp*. lacti* HOM1119 strain can achieve the function of lowering blood glucose level by producing higher levels of DPP-4 inhibitor and α-glucosidase inhibitor.

### Example 9. Stimulation secretion of GLP-1 in NCI-H716 cells by B. animalis subsp. lacti HOM1119 strain

### (1) Culture of NCI-H716 Cells

In this experiment, NCI-H716 cells (purchased from Cell Bank, Chinese Academy of Sciences) were selected. NCI-H716 cells were grown in suspension in RPMI-1640 (Gibco) medium containing 10% fetal bovine serum (Hyclone), 1% double-antibody (penicillin and streptomycin, Hyclone) at 37°C in an 5% CO₂ incubator.

### (2) Culture of Strains

A commercial strain *L. rhamnosus* GG(ATCC53103) strain (purchased from China Center of Industrial Culture Collection) and *B. animalis* subsp*. lactis* BH13-25 and BH13-33 strains were selected as control strains. The *B. animalis* subsp*. lactis* HOM1119, BH13-25 and BH13-33 strains and *L. rhamnosus* GG strain were inoculated into the sterilized MRS-Cys liquid medium described in Example 1, respectively, at an inoculation amount of 1% of the total amount of the medium, statically cultured under anaerobic conditions at 37°C for 24 hours and activated twice, and then the strain fermentation solution was obtained. Then the strain fermentation solution was centrifuged at 8000 rpm for 10 min, and the strain concentration was adjusted to 1× 10¹⁰ CFU/mL with a sterile 0.9% sodium chloride solution. The strain fermentation solution was anaerobically cultured for 8 h, centrifuged at 8000 rpm for 10 min, and the supernatant was collected for later use.

### (3) GLP-1 Endocrine Experiment

NCI-H716 cells were seeded at a density of 1.5 × 10 ⁶ cells/well in a 24-well plate coated with matrigel. Endocrine differentiation medium was added, and the plate was incubated at 37°C in 5% CO₂ incubator for 2 days for endocrine differentiation experiments. The endocrine differentiation medium was a high-glucose DMEM (Gibco) medium containing 10% fetal bovine serum, and 1% the double-antibody.

After 2 days, the DMEM medium was replaced with Krebs-Ringer buffer. The buffer contained 1 × 10¹⁰ CFU/mL of probiotic strains, respectively. After culturing for 2 h, it was centrifuged at 8000 rpm for 10 min, and the supernatant was collected. 50µg/mL of phenylmethylsulfonyl fluoride and 10µg/mL of sitagliptin were added to the supernatant, and then the concentration of GLP-1 was detected by ELISA kit (Ray biotech). Each treatment was performed in triplicate. The data were analyzed using SPSS 25.0 software for one-way ANOVA and Duncan's multiple comparisons. The results were shown in Table 9.

**Table 9. The yield of GLP-1 secretion in NCI-H716 cells stimulated by B. animalis subsp. lacti HOM1119 strain ( x ± SD)**

| Strain Name | GLP-1(pg/mL) |
|---|---|
| HOM1119 | 2413.02±60.67^{A} |
| GG | 962.34±44.62^{B} |
| HOM13-33 | 765.1±22.63^{C} |
| HOM13-25 | 639.39±20.62^{D} |

| | |
|---|---|
| Note: In the same column, different capital letters for the superscript indicate highly significant differences (p < 0.01). | |

It can be seen from Table 9 that, compared with the control strains, *B. animalis* subsp*. lactis* BH13-25, BH13-33 and *L. rhamnosus* GG, the *B. animalis* subsp*. lactis* HOM1119 strains stimulated NCI-H716 cells to secrete a high content of GLP-1, reaching the concentration of 2413.02 pg/mL, and the difference was very significant (p < 0.01). It indicated that *B. animalis* subsp*. lacti* HOM1119 strain has the effect of lowering blood glucose, and the mechanism of lowering blood glucose may be stimulating intestinal L cells to produce high levels of GLP-1.

### Example 10. In Vitro Test 1 for Inhibition of Lipid Synthesis by B. animalis subsp. lacti HOM1119 strain

### (1) Preparation of Cell Medium

Complete medium 1: Into high-sugar DMEM medium, 10% of inactivated new bovine serum (NBS) and 1% (v/v) of antibiotic (100 U/mL penicillin, 100 µg/mL streptomycin) were added, mixed and then stored at 4 °C.

Complete medium 2: Into high-sugar DMEM medium, 10% inactivated fetal bovine serum (FBS) was added, mixed and stored at 4 °C.

Cell differentiation medium 1: 3-isobutyl 1-methylxanthine (IBMX) was added to complete medium 2 to achieve a final concentration of 0.5 mol/L, dexamethasone (DEX) was added to achieve a final concentration of 1µmol/L, and insulin was added to achieve a final concentration of 10µg/mL.

Cell differentiation medium 2: Insulin was added to complete medium 2 to achieve a final concentration of 10µg/mL, and stored at 4°C for later use.

### (2) Preparation of Test Cell Samples

The *B. animalis subsp. lacti* HOM1119 strain was inoculated into sterilized MRS-Cys liquid medium as described in Example 1 at an inoculum amount of 1% of the total culture medium, statically cultured under aerobic conditions at 37°C for 24 hours. After being activated two generations, the bacterial cells were collected by centrifugation at 8000 r/min for 10 min, washed twice with PBS, and resuspended in PBS solution (0.1 mol/L, pH 7.2 to 7.4) to form a bacterial suspension. The bacterial suspension was placed in a water bath, treated at 70°C for 30 minutes to obtain heat-killed bacteria, and stored at - 80°C for later use.

### (3) Culture of 3T3-L1 preadipocytes

3T3-L1 preadipocytes (purchased from Institute of Basic Medical Sciences, Chinese Academy of Medical Sciences) were inoculated into complete medium 1 at a ratio of 1:3 for passage, and 3-10 passages of cells were selected for testing.

### (4) Assay of Cell Viability

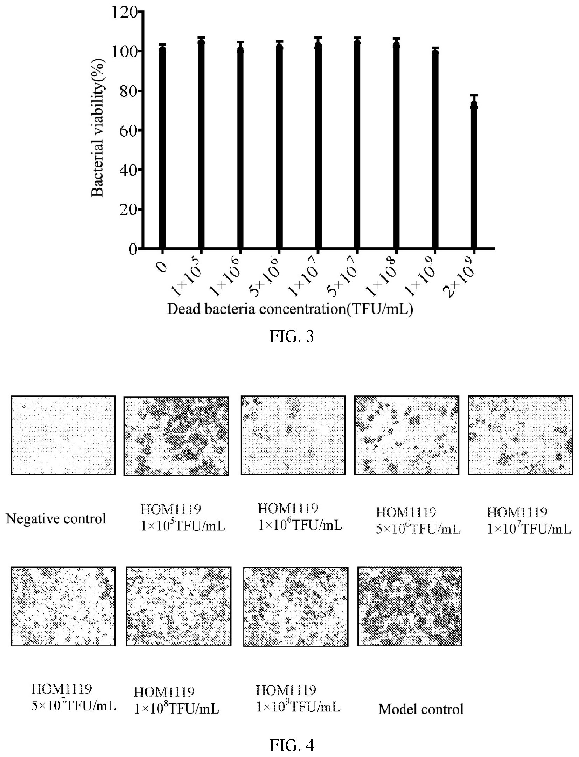

The cell count was adjusted to 2 × 10⁵ cells/mL with complete medium 2. 0.1 mL of cell solution was pipetted into each well and seeded into a 96-well culture plate, cultured for 2 days for later use. The cultured 96-well plate cells were taken, the cell culture medium was aspirated, 0.1 mL of different concentrations of test cells (adjusted with complete medium 2) were added to each well, cultured in a 5% CO₂ incubator at 37°C for 24 h. The cell viability was detected using the MTS cell proliferation and cytotoxicity assay kit manufactured by Promega. The simplified procedure was as follows: 20µL of MTS was added to each well, incubated in an incubator in the dark for 1h, and the absorbance was read at 490 nm using a microplate reader. Each treatment used cell culture medium 2 + test cells + MTS as blank control. Complete medium 2 + cells + MTS was used as negative control. The test results are shown in FIG. 3.

FIG. 3 shows the effect of *B. animalis subsp. lacti* HOM1119 strain on 3T3-L1 preadipocytes viability.

It can be seen from the results in FIG. 3 that when the concentration of dead bacteria was not greater than 1 × 10⁸ TFU/mL, the viability of 3T3-L1 preadipocytes was higher than 100%. Therefore, 1 × 10⁵ TFU/mL, 1 × 10⁶ TFU/mL, 5 × 10⁶ TFU/mL, 1 × 10⁷ TFU/mL, 5 × 10⁷ TFU/mL, 1 × 10⁸ TFU/mL and 1 × 10⁹ TFU/mL were selected for the evaluation of the lipid lowering effect of cells.

### (5) 3T3-L1 Preadipocytes Differentiation

The cultured cells were inoculated with 2 × 10⁵ cells/mL of complete medium at 1 mL per well into a 24-well culture plate coated with 0.1% gelatin. 3T3-L1 preadipocytes were cultured until reaching 100% confluence. Three days after confluence was recorded as day 0. Cell differentiation medium 1 was added, and the cells were cultured for 4 days, recorded as day 4. Then, cell differentiation medium 2 was replaced for induction, and the cells were cultured for 2 days, recorded as day 6. After induction differentiation, the cells were cultured in complete medium 2 for 2 days, recorded as day 8. On day 8, 3T3-L1 preadipocytes were differentiated into round mature adipocytes with round triglyceride particles inside, during which the medium was changed every 2 days. The bacterial samples were added at day 0, bacterial samples with different concentrations showing cell viability higher than 100% were added, and co-cultured with 3T3-L1 cells from day 0 to day 8 of induced differentiation.

### (6) Oil Red O Staining

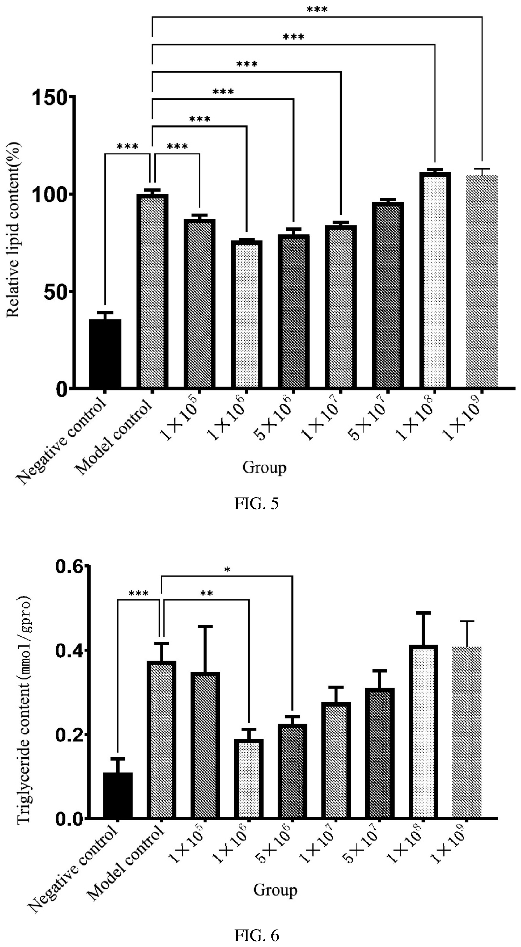

The differentiated mature adipocytes were gently washed 3 times with an appropriate amount of PBS, fixed with 0.5 mL of 4% paraformaldehyde for 30 min, and then gently washed 3 times with ultrapure water. 0.5 mL of Oil Red O working solution was added to each well, and stained at 37°C for 30 min in the dark. The staining solution was discarded, and then gently washed 3 times with ultrapure water for 3 times. The culture plate was placed under an inverted microscope for observation, and photographed. After the photographing, an equal amount of 1 mL of 100% isopropanol was added to each well to extract Oil Red O staining solution from the lipid droplets, placed at room temperature for 20 min. The extracted staining solution was pipetted and transferred to a clean 96-well culture plate. The absorbance value was measured at 500 nm. Absorbance values were determined at 500 nm, using 100% isopropanol as a blank. The relative lipid content was calculated as follows: relative lipid content (%) = (ODₛₐₘₚₗₑ)/OD_{blank}) × 100%. Each treatment was performed in triplicate. The data were analyzed by using GraphPad prism9 software for one-way ANOVA and Dunnett's t test, i.e., pairwise comparison and analysis of the means between multiple experimental groups and a control group. The results of oil staining and lipid content were shown in FIGS. 4 and 5.

3T3-L1 cells aggregated a large number of lipid droplets in the cells after differentiation and maturation. Oil Red O staining can visually reflect the amount of lipid accumulation after adipogenesis. It can be seen from FIG. 6 that 90% of the cells in the model group were differentiated into mature adipocytes on the day 8 of induction, which showed that the cells were further enlarged, round in shape, and filled with red lipid droplets densely clustered around the nucleus, forming a "ring-like" structure in the form of typical mature fat. Compared with the model control group, after 8 days of induction differentiation of preadipocytes treated with dead *B. animalis* subsp*. lactis* HOM1119 strain, all four concentrations of 1 × 10⁷ TFU/mL, 5 × 10⁶ TFU/mL, 1 × 10⁶ TFU/mL and 1 × 10⁵ TFU/mL can significantly reduce the number of intracellular lipid droplets, showing a dose-dependent effect. The best effect was at a concentration of 1 × 10⁶ TFU/mL. The quantitative results of Oil Red O dye of adipocytes (FIG. 5) showed that the cell-free extract of *B. animalis* subsp*. lactis* HOM1119 strain can significantly reduce the intracellular lipid content (p < 0.01), showing a dose-dependent effect. With the increase of the cell-free extract concentration of *B. animalis* subsp*. lactis* HOM1119 strain, its ability to inhibit 3T3-L1 preadipocytes differentiation was first enhanced and then reduced. When the cell-free extraction concentration of *B. animalis* subsp*. lactis* HOM1119 was 1 × 10⁶ TFU/mL, the intracellular relative lipid content was the lowest, and the weight loss effect was the best.

### (6) Determination of Intracellular Triglyceride (TG) Content in 3T3-L1

The intracellular triglyceride content was determined according to the instructions of the triglyceride (TG) assay kit produced by Nanjing Jiancheng Biotechnology Research Institute. The simplified steps were as follows: 2.5µL of sample and 250µL of working solution were added to each well, mixed well, incubated at 37°C for 10 minutes, and the absorbance was read at 510 nm using a microplate reader. Protein concentrations were determined using a total protein quantification kit (BCA method). The simplified steps were as follows: 10µL of sample and 250µL of working solution were added to each well, mixed well, incubated at 37°C for 10 minutes, and the absorbance was read at 562 nm using a microplate reader. The glyceride content per gram of protein was calculated according to the formula. Calculation formual: $\begin{matrix}Triglycerides \left(mmol/gprot\right) \\ = \frac{Sample OD value - Blank OD value}{Standard OD value - Blank OD value} \times Calibrator concentration \left(mmol/L\right) \div Protein concentration \\ of sample to be tested \left(gprot/L\right)\end{matrix}$

Each treatment was performed in triplicate. The data were analyzed by using GraphPad prism9 software for one-way ANOVA and Dunnett's t test, i.e., pairwise comparison and analysis of the means between multiple experimental groups and a control group. The intracellular triglyceride (TG) content of 3T3-L1 cells was shown in Figure 6. With the increase of the cell-free extract concentration of *B. animalis* subsp. *lactis* HOM1119 strain, the triglyceride (TG) content in 3T3-L1 preadipocyte initially decreased and then increased. When the concentration of dead bacteria in *B. animalis* subsp. *lactis* HOM1119 strain was 1 × 10⁶ TFU/mL, the intracellular lipid content can be significantly reduced (p < 0.01), and when the concentration was 5 × 10⁶ TFU/mL, the triglyceride (TG) content in 3T3-L1 cells can be significantly reduced (p < 0.05). It indicated that *B. animalis* subsp. *lactis* HOM1119 strain has a weight loss function.

### Example 11. In Vitro Test 2 for Inhibition of Lipid Synthesis by B. animalis subsp. lacti HOM1119

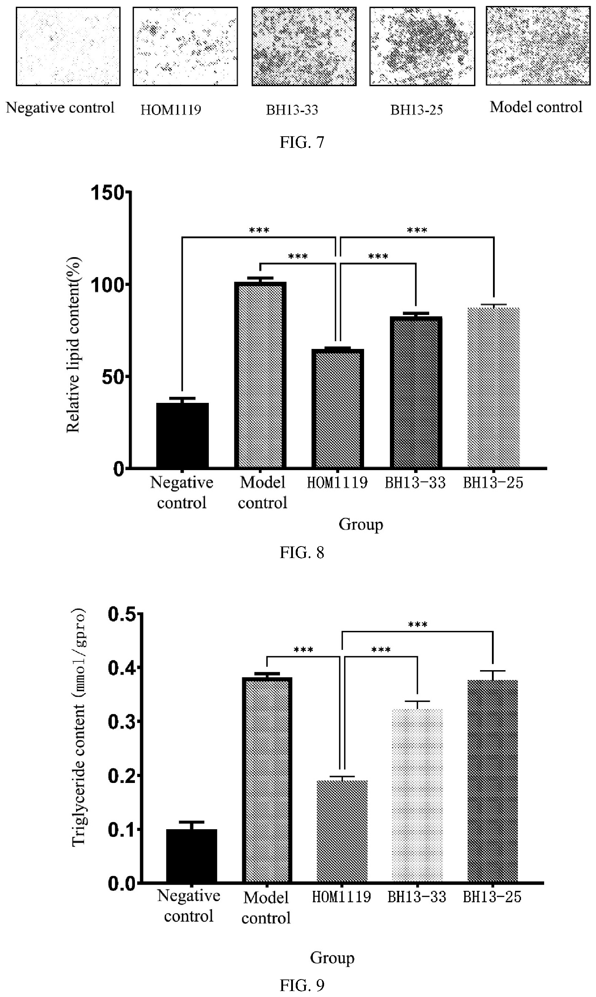

According to the method of Example 10, *B. animalis subsp. lacti* BH13-25 and BH13-33 strains were used as control strains, and a dead bacterial concentration of 1 × 10⁶ TFU/mL was selected for the evaluation experiment of the cell lipid-lowering effect. The results of oil staining and lipid content were shown in FIGS. 7 and 8, and the content of intracellular triglyceride (TG) of 3T3-L1 cells was shown in FIG. 9. It can be seen that, compared with the control strains *B. animalis* subsp. *lactis* BH13-25 and BH13-33, the *B. animalis* subsp. *lactis* HOM1119 strain can significantly reduce the intracellular lipid content and triglyceride (TG) content (p < 0.01).

### Example 12. Preparation Process of Active Bacterial Powder of B. animalis subsp. lacti HOM1119 Strain

### (1) Culture of Strain

The *B. animalis* subsp*. lactis* HOM1119 strain cryopreserved at - 80°C was inoculated into the sterilized MRS-Cys liquid medium at an inoculation amount of 1%, and cultured at 37°C for 16-24 hours. After two such subcultures, activated seed culture solution was obtained. The seed culture solution was inoculated into a fermentation medium M473 at an inoculation amount of 1%, and cultured anaerobically at constant temperature of 35-37°C with a rotation speed of 100 rpm and a nitrogen pressure of 200-500 mbar. During the fermentation, a sodium hydroxide solution was automatically added to maintain the pH constant at 5.0-5.5. After fermentation for 16-24 hours, it was transferred to another tank to obtain a high-density culture solution of *B. animalis* subsp*. lactis* HOM1119, with a viable count of up to 150-200 billion CFU/mL. Table 10 showed the formulation of fermentation medium M473.

**Table 10. The formulation of Fermentation medium M473**

| Medium Components | Content (g/L) |
|---|---|
| Maltose | 40 |
| Yeast Extract | 50 |
| Magnesium Sulfate | 0.4 |
| Manganese Sulfate | 0.2 |
| Dipotassium hydrogen phosphate | 4 |
| Potassium Dihydrogen Phosphate | 4 |
| Tween-80 | 1 |
| Calcium Chloride | 0.1 |
| L-Cysteine Salt | 1 |

### (2) Preparation of Lyoprotectant

A protectant containing 100-150 g/L of modified starch, 20-50 g/L of trehalose, 0.2-1 g/L of vitamin C and 0.2-1 g/L of glycerol was prepared by mixing sterile water with raw materials of the protectant.

### (3) Freeze-Drying

The fermentation solution of *B. animalis* subsp*. lactis* HOM1119 after the culture was centrifuged at 6500 rpm for 15 min at 2-8°C. The supernatant was discarded, the bacterial sludge was collected. The bacterial sludge was washed with 0.9% sterile physiological saline for 1-2 times. The washed bacterial sludge was mixed with the above-mentioned protectant, the concentration of the strain reached 10¹⁰ CFU/mL or more in the mixed bacterial solution. It was then freeze-dried in a freeze dryer, pre-frozen at -50°C for 4 hours, vacuumized, the temperature was raised to -20 °C, and dried for 40 hours in a primary drying; then the temperature was raised to 35°C, and dried for 6 hours in a secondary drying. After freeze-drying, the bacterial cake was pulverized by a fine grinder to obtain the freeze-dried bacterial powder. The viable count of the freeze-dried bacterial powder reached 5~7 × 10¹¹ CFU/g.

### Example 13. Effect of B. animalis subsp. lactis HOM1119 strain on blood glucose in model rats with insulin resistant glucose/lipid metabolism disorder induced by alloxan

(1) 55 healthy SPF-grade male SD mice (140 g -160 g), bred by Beijing Huafukang Biotechnology Co., Ltd [License No.: SCXK-( 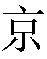 )2019-0008], were selected. The experimental animals were housed in the SPF-grade animal room of the Health Food Function Detection Center, the College of Art and Science, Beijing Union University, and the experimental animal used license number: SYXK( 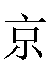 )2017-0038. The experiment was performed in two batches. The first batch of experiment was performed with 10 rats to test the effect on fasting blood glucose in normal rats; the second batch of experiment was performed with 45 rats to test fasting blood glucose in model rats with insulin resistant glucose/lipid metabolism disorder induced by alloxan, a glucose tolerance experiment, a serum triglyceride and total cholesterol experiment, and a serum insulin experiment. The formula of the high-energy feed was: 52.6% of basic feed, 15% of sucrose, 15% of egg yolk powder, 10% of lard, 5% of casein, 1.2% of cholesterol, 0.2% of sodium cholate, 0.6% of calcium bicarbonate, and 0.4% of mineral powder.
(2) Experiment on the effect of fasting blood glucose in normal rats: After acclimatization to a normal diet for 3 days, the rats were fasted for 4 h. Blood was collected, allowed to stand for 10 min, and centrifuged at 3000 r/min for 10 min to separate serum. Blood glucose level before glucose administration (i.e., 0 h) and 0.5 and 2 h after administration of 2.5 g/kg body weight (BW) glucose were measured by using a biochemical analyzer, which was used as the basic value of this batch of animals. A normal control group (0 CFU/rat) and HOM1119 group (5 × 10¹⁰ CFU/kg BW) were set; each group consisted of 5 rats. At the end of the experiment, rats in each group were fasted for 4 h and blood was collected from the venous plexus of angular vein of eyes. The fasting blood glucose level was detected using a biochemical analyzer, and the body weights of the rats at the initial and at the end were determined.
(3) Fasting blood glucose experiment in model rats with insulin resistant glucose/lipid metabolism disorder induced by alloxan: A blank control group, a model group and a HOM1119 strain group were set up, with 15 rats in each group. The HOM1119 group was administered at a dose of 5 × 10¹⁰ CFU/kg BW via gavage. 0.5 mL test substance was administered to each animal by gavage, while the blank control group and the model control group were administered with the same volume of physiological saline, once daily by gavage. After one week of feeding with basic feed for each group, the model control group and the HOM1119 group were replaced with high-energy feed. After three weeks of continued feeding for each group, the model control group and the HOM1119 group were fasted for 24 h, and then intraperitoneally injected with alloxan at 105 mg/kg BW, with injection volume of 1 mL/100g body weight. The high-energy feed feeding was continued for 5 days after injection. At the end of the experiment, rats in each group were fasted for 4 h, blood was collected to detect fasting blood glucose, glucose tolerance, serum insulin, total cholesterol and triglyceride levels.
(4) Data processing: SPSS software was used for data processing. Independent sample t-test was used to analyze all indicators in the blank control group and the model control group, and the model control group and the test group.

**Table 11. Body weight ( x±SD) of normal rats before and after the experiment**

| Group | Number of Rats | Before Experiment | | After Experiment | |
|---|---|---|---|---|---|
| | | Body Weight (g) | P Value | Body Weight (g) | P Value |
| Normal Control Group | 5 | 237.9±21.0 | - | 442.5±22.6 | - |
| HOM1119 Group | 5 | 239.9±7.7 | 0.939 | 462.2±38.0 | 0.912 |

It can be seen from Table 11 that the weight of normal rats before and after the experiment was compared between the two groups, and the difference was not significant (p > 0.05).

**Table 12. The effects of B. animalis subsp. lactis HOM1119 strain on fasting blood glucose in normal rats ( x ± SD)**

| Group | Number of Rats (n) | Blood Glucose (mmol/L) | | | | Percent Decrease (%) | P Value |
|---|---|---|---|---|---|---|---|
| | | Before Experiment | P Value | After Experiment | P Value | | |
| Normal Control Group | 5 | 7.4±0.7 | - | 7.4±0.3 | | 0.3±8.6 | - |
| HOM1119 Group | 5 | 7.5±0.4 | 0.684 | 7.1±0.2 | 0.115 | 5.5±6.8 | 0.684 |

It can be seen from Table 12 that there were no significant differences (p > 0.05) in blood glucose levels and the percentage decrease in blood glucose between the normal animal dose groups and the normal control group before the experiment and after the administration of the test substance of normal rats.

**Table 13. The body weights of rats in the blank and model control groups**

| Group | Number of Rats | Before Experiment | | After Experiment | |
|---|---|---|---|---|---|
| | | Body Weight (g) | P Value | Body Weight (g) | P Value |
| Blank Control Group | 15 | 236.4±15.2 | - | 460.4±45.6 | - |
| Model Control Group | 15 | 241.1±15.2 | 0.402 | 465.6±42.5 | 0.750 |

It can be seen from Table 13 that, there was no significant difference in body weight of rats before and after the experiment between the model control group and the blank control group (p > 0.05).

**Table 14. Blood glucose and glucose tolerance in a rat model of lipid metabolism disorder ( x ± SD).**

| Group | Number of Rats | Blood Glucose Level After Glucose Administration | | | | | | | |
|---|---|---|---|---|---|---|---|---|---|
| | | 0 h | P Value | 0.5 h | P Value | 2 h | P Value | Area Under the Curve | P Value |
| Blank Control Group | 15 | 7.4±0.6 | - | 8.8±1.0 | - | 7.0±1.2 | - | 15.9±1.6 | - |
| Model Control Group | 15 | 8.4±0.5** | 0.000 | 10.2±1.5** | 0.005 | 7.7±0.9 | 0.089 | 18.1±2.0** | 0.003 |

| | | | | | | | | | |
|---|---|---|---|---|---|---|---|---|---|
| **: There was significant difference compared with the blank control group (p < 0.01) | | | | | | | | | |

**Table 15. Blood lipid and insulin resistance index of rat model with glucose and lipid metabolism disorder ( x ± SD)**

| Group | Number of Rats | Total Cholesterol (mmol/L) | P Value | Triglyceride (mmol/L) | P Value | Insulin Resistance Index | P Value |
|---|---|---|---|---|---|---|---|
| Blank Control Group | 15 | 2.0±0.2 | - | 1.6±0.6 | - | 2.78±0.47 | - |
| Model Control Group | 15 | 2.4±0.5* | 0.032 | 1.7±0.5 | 0.634 | 3.08±0.28* | 0.048 |

| | | | | | | | |
|---|---|---|---|---|---|---|---|
| *: There was significant difference compared with the blank control group (p < 0.05) | | | | | | | |

It can be seen from Tables 14 and 15 that, compared with the blank control group, the model control group showed highly significant differences in the blood glucose levels and the area increase under the blood glucose curve at 0 h and 0.5 h after glucose administration (p < 0.01). The blood glucose level in the model control group at 0.5 h was ≥ 10 mmol/L, indicating that the glucose metabolism disorder model was established. Comparing with the blank control group, the model control group showed a significant difference in the serum total cholesterol increase (p < 0.05), confirming the lipid metabolism disorder model was established. Comparing with the blank control group, the model control group showed a significant decrease in the insulin resistance index (p < 0.05), and it was comprehensively confirmed that the successful establishment of model with insulin resistance glucose/lipid metabolism disorder.

**Table 16. The effects of B. animalis subsp. lactis HOM1119 strain on body weight of rats in model rats with insulin resistance glucose/lipid metabolism disorder ( x ± SD).**

| Group | Number of Rats (n) | Before Experiment | | After Experiment | |
|---|---|---|---|---|---|
| | | Body Weight (g) | P Value | Body Weight (g) | P Value |
| Model Control Group | 15 | 241.1±15.2 | - | 465.6±42.5 | - |
| HOM1119 Group | 15 | 242.6±11.9 | 0.773 | 459.6±31.5 | 0.688 |

It can be seen from Table 16 that, after oral administration of different probiotic powders to the model rats, there was no significant difference in the body weights of the rats between each group and the model control group (p > 0.05).

**Table 17. Effect of B. animalis subsp. lactis HOM1119 strain on fasting blood glucose in model rats with insulin resistant glucose/lipid metabolism disorder ( x ± SD)**

| Group | Number of Rats (n) | Blood Glucose (mmol/L) | | | | Percent Decrease (%) | P Value |
|---|---|---|---|---|---|---|---|
| | | Before Experiment | P Value | After Experiment | P Value | | |
| Model Control Group | 15 | 7.1±0.9 | - | 8.4±0.5 | - | -18.9±14.9 | - |
| HOM1119 Group | 15 | 7.1±0.8 | 0.899 | 8.0±0.4* | 0.018 | -13.2±12.5 | 0.264 |

| | | | | | | | |
|---|---|---|---|---|---|---|---|
| *: There was significant difference compared with the model control group (p < 0.05) | | | | | | | |

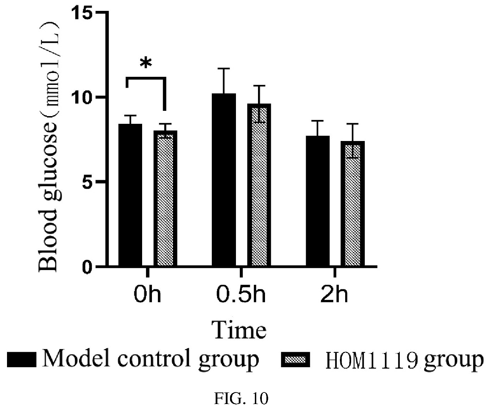

It can be seen from Table 17 and FIG. 10 that after oral administration of the *B. animalis* subsp. *lactis* HOM1119 strain to the model rats with insulin resistance to glucose/lipid metabolism disorder, compared with the model control group, the fasting blood glucose decreased, and there was a significant difference (p < 0.05).

**Table 18. The effects of B. animalis subsp. lactis HOM1119 strain on the glucose tolerance of model rats with insulin resistant glucose/lipid metabolism disorder ( x ± SD).**

| Group | Number of Rats | Blood Glucose Level After Glucose Administration | | | | | |
|---|---|---|---|---|---|---|---|
| | | 0h | P Value | 0.5 h | P Value | 2h | P Value |
| Model Control Group | 15 | 8.4±0.5 | - | 10.2±1.5 | - | 7.7±0.9 | - |
| HOM1119 Group | 15 | 8.0±0.4 | 0.018 | 9.6±1.1 | 0.182 | 7.4±1.0 | 0.373 |

It can be seen from Table 18 and FIG. 10 that after oral administration of the *B. animalis* subsp*. lactis* HOM1119 strain to the model rats with insulin resistant glucose/lipid metabolism disorder, there was no significant differences in blood glucose levels at 0.5h and 2 h after glucose administration compared to the model control group (p > 0.05).

**Table 19. Effect of B. animalis subsp. lactis HOM1119 strain on the area under the blood glucose curve of rats in the model rats with insulin resistant glucose/lipid metabolism disorder ( x ± SD).**

| Group | Number of Rats | Area Under Blood Glucose Curve | P Value |
|---|---|---|---|
| Model Control Group | 15 | 18.1±2.0 | - |
| HOM1119 Group | 15 | 17.1±1.5 | 0.128 |

It can be seen from Table 19 that after oral administration of *B. animalis* subsp*. lactis* HOM1119 strain to rats, there was no significant difference in the area under the blood glucose curve at 0-2h after administration of glucose compared with the model control group (p > 0.05).

**Table 20. Effects of B. animalis subsp. lactis HOM1119 strain on serum cholesterol and triglyceride in model rats with insulin resistant glucose/lipid metabolism disorder ( x ± SD)**

| Group | Number of Rats | Serum Cholesterol (mmol/L) | P Value | Triglyceride (mmol/L) | P Value |
|---|---|---|---|---|---|
| Model Control Group | 15 | 2.4±0.5 | - | 1.7±0.5 | - |
| HOM1119 Group | 15 | 2.3±0.3 | 0.660 | 1.6±0.4 | 0.392 |

It can be seen from Table 20 that after oral administration of *B. animalis* subsp*. lactis* HOM1119 strain to rats, there was no significant difference in serum total cholesterol and serum triglyceride compared with the model control group (p > 0.05).

**Table 21. Effect of B. animalis subsp. lacti HOM1119 strain on serum insulin resistance index of model rats with insulin resistant glucose/lipid metabolism disorder ( x ± SD)**

| Group | Number of Rats | Insulin Resistance Index | P Value |
|---|---|---|---|
| Model Control Group | 12 | 3.08±0.28 | - |
| HOM1119 Group | 12 | 2.95±0.41 | 0.340 |

It can be seen from Table 21 that after oral administration of *B. animalis* subsp*. lactis* HOM1119 strain to rats, there was no significant difference in the serum insulin resistance index compared with the control group (p > 0.05).

In summary, after oral administration of *B. animalis* subsp*. lactis* HOM1119 strain to the model rats with insulin resistant glucose/lipid metabolism disorder, compared with the model control group (0 g/kg BW), the fasting blood glucose of the *B. animalis* subsp*. lactis* HOM1119 strain group decreased significantly (p < 0.05), while glucose tolerance showed no significant difference (p > 0.05), and blood lipid (total cholesterol and triglycerides) of rats did not increase significantly. The tested substance, *B. animalis* subsp*. lactis* HOM1119 strain, has no adverse effects on fasting blood glucose and body weight in normal rats. Therefore, it is believed that *B. animalis* subsp*. lactis* HOM1119 strain has an auxiliary function in lowering blood sugar.

The above description is only preferred embodiments of the present disclosure, and it should be noted that for those skilled in the art, several improvements and modifications can be made without departing from the principle of the present disclosure, and these improvements and modifications should also be considered within the protection scope of the present invention.

Although the embodiments of the present invention have been disclosed as above, they are not limited to the applications listed in the specification and the embodiments. They can be fully applied to various fields suitable for the present invention, and those skilled in the art can easily realize other modifications. Therefore, without departing from the general concept defined by the claims and the equivalent scope, the present invention is not limited to specific details and the details shown and described herein.

## Claims

1. A *Bifidobacterium animalis* subsp*. lacti* (*B. animalis* subsp*. lacti*) strain, wherein the *B. animalis* subsp*. lacti* strain has a deposit number of CGMCC No. 25680.

2. The *B. animalis* subsp*. lacti* strain according to claim 1, wherein the *B. animalis* subsp*. lacti* strain comprises a 16S rRNA gene represented by SEQ ID NO: 1.

3. The *B. animalis* subsp*. lacti* strain according to claim 1 or 2, which has the ability to produce high levels of lactic acid and short-chain fatty acid.

4. The *B. animalis* subsp*. lacti* strain according to claim 3, wherein the short-chain fatty acid is selected from the group consisting of one or more of formic acid, acetic acid, propionic acid, butyric acid and isobutyric acid.

5. The *B. animalis* subsp*. lacti* strain according to claim 1 or 2, which has antioxidant activity.

6. The *B. animalis* subsp*. lacti* strain according to claim 5, wherein the *B. animalis* subsp*. lacti* strain has hydroxyl radical scavenging and DPPH radical scavenging activities, and has SOD enzyme activity.

7. A probiotic preparation for facilitating weight loss or assisting in lowering blood glucose, comprising the *B. animalis* subsp*. lacti* strain according to claim 1 or 2 and excipients.

8. The probiotic preparation according to claim 7, wherein the probiotic preparation is a viable bacteria preparation, and the excipient is selected from the group consisting of one or more of starch, sucrose, trehalose and glycerol.

9. The probiotic formulation of claim 7, wherein the viable bacterial preparation comprises up to 5.0-7.0 × 10¹¹ CFU/g of viable bacteria.

10. A method for preparing the probiotic preparation according to any one of claims 7 to 9, comprising the following steps: expanding the culture of the *B. animalis* subsp*. lacti* strain according to claim 1 in an optimized liquid medium; collecting the bacteria; adding a protectant for resuspension, freeze-drying under vacuum, and pulverizing to obtain an active bacterial preparation.

11. Use of the *B. animalis* subsp*. lacti* strain according to claim 1 or 2 in the preparation of a medicament for weight loss.

12. The use according to claim 11, wherein the *B. animalis* subsp*. lacti* strain is used to inhibit differentiation of 3T3-L1 preadipocytes.

13. The use according to claim 11, wherein the *B. animalis* subsp*. lacti* strain is used to reduce the triglyceride (TG) content in 3T3-L1 cells.

14. The use according to claim 11, wherein the *B. animalis* subsp*. lacti* strain is used to inhibit fat production.

15. The use according to claim 11, wherein the *B. animalis* subsp*. lacti* strain is used to treat metabolic diseases caused by obesity.

16. Use of the *B. animalis* subsp*. lacti* strain according to claim 1 or 2 in the preparation of a pharmaceutical composition for preventing or treating obesity and related diseases.

17. Use of the *B. animalis* subsp*. lacti* strain according to claim 1 or 2 in the preparation of a medicament for assisting in lowering blood glucose.

18. The use according to claim 17, wherein the *B. animalis* subsp*. lacti* strain is used to inhibit the activity of dipeptidyl peptidase 4 (DPP-4) and α-glucosidase.

19. The use according to claim 17, wherein the *B. animalis* subsp*. lacti* strain is used to stimulate the secretion of GLP-1 by NCI-H716 cells.

20. Use of the *B. animalis* subsp*. lacti* strain according to claim 1 or 2 in the preparation of a pharmaceutical composition for preventing or treating diabetes.

21. A food or health product comprising the *B. animalis* subsp*. lacti* strain according to claim 1 or 2.

22. A pharmaceutical composition comprising the *B. animalis* subsp*. lacti* strain according to claim 1 or 2 and a pharmaceutically acceptable excipient.
